(19) Europäisches Patentamt
European Patent Office
Office européen des brevets

(11) Veröffentlichungsnummer: **0 009 740**
**B1**

(12) **EUROPÄISCHE PATENTSCHRIFT**

(45) Veröffentlichungstag der Patentschrift: **20.10.82**

(51) Int. Cl.[3]: **C 07 D 249/08,** C 07 D 405/06, C 07 D 409/06, A 01 N 43/64

(21) Anmeldenummer: **79103583.5**

(22) Anmeldetag: **24.09.79**

(54) **Beta-Triazolyloxime, diese enthaltende Mittel zur Beeinflussung des Pflanzenwachstums und ihre Anwendung.**

(30) Priorität: **30.09.78 DE 2842801**

(43) Veröffentlichungstag der Anmeldung: **16.04.80 Patentblatt 80/8**

(45) Bekanntmachung des Hinweises auf die Patenterteilung: **20.10.82 Patentblatt 82/42**

(84) Benannte Vertragsstaaten: **AT BE CH DE FR GB IT LU NL SE**

(56) Entgegenhaltungen:
**EP-A-0 000 940**
**DE-A-2 634 511**
**DE-A-2 704 682**
**DE-A-2 734 107**
**DE-A-2 739 352**

(73) Patentinhaber: **BASF Aktiengesellschaft, Carl-Bosch-Strasse 38, D-6700 Ludwigshafen (DE)**

(72) Erfinder: **Rentzea, Costin, Dr., Neuenheimer Landstrasse 72, D-6900 Heidelberg (DE)**
Erfinder: **Sauter, Hubert, Dr., Goethestrasse 23, D-6700 Ludwigshafen (DE)**
Erfinder: **Zeeh, Bernd, Dr., Thorwaldsenstrasse 5, D-6700 Ludwigshafen (DE)**
Erfinder: **Heilen, Gerd, Dr., Schifferstadter Strasse 1 b, D-6720 Speyer (DE)**
Erfinder: **Jung, Johann, Dr., Hardenburgstrasse 19, D-6703 Limburgerhof (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

# β-Triazolyloxime, diese enthaltende Mittel zur Beeinflussung des Pflanzenwachstums und ihre Anwendung

Die vorliegende Erfindung betrifft neue wertvolle substituierte β-Triazolyloxime, Verfahren zu ihrer Herstellung, Mittel zur Regulierung des Pflanzenwachstums, die diese Verbindungen enthalten, und Verfahren zur Regulierung des Pflanzenwachstums mit diesen Verbindungen.

Es ist bereits bekannt, das 2-Chloräthyl-trimethylammoniumchlorid (Chlorcholinchlorid, CCC) [J. Biol. Chem., 235, 475 (1960)] zur Beeinflussung des Pflanzenwachstums zu verwenden. Mit seiner Hilfe lässt sich z.B. eine Hemmung des Längenwachstums bei einigen Getreidearten und eine Hemmung des vegetativen Wachstums bei einigen anderen Kulturpflanzen erreichen. Die Wirkung dieses Stoffes ist jedoch, insbesondere bei niedrigen Aufwandmengen, nicht immer ausreichend und genügt den Anforderungen der Praxis nicht immer.

Weiterhin ist es bekannt, 1-(4′-Bromphenyl)-1-allyloxy-2-[1″, 2″, 4″-triazolyl-(1″)]-äthan zur Regulierung des Pflanzenwachstums von Raps, Weizen, Hafer, Roggen und Gerste zu verwenden (Deutsche Offenlegungsschrift 2 650 831). Dessen Wirkung ist jedoch, vor allem bei niedrigen Aufwandmengen, nicht immer befriedigend.

Es wurde nun gefunden, dass Verbindungen der Formel

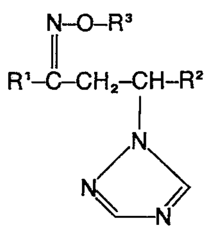

I

in der

$R^1$ und $R^2$ verschieden oder gleich sind und Alkyl mit 1 bis 6 C-Atomen, Furanyl, Thiophenyl, Naphthyl, Biphenylyl oder Phenyl bedeuten, wobei der Phenylrest durch Fluor, Chlor, Brom, Alkyl mit 1 bis 4 C-Atomen, Trifluormethyl, Nitro oder Alkoxy mit 1 bis 4 C-Atomen substituiert sein kann und $R^3$ ein Wasserstoffatom, einen Alkylrest mit 1 bis 4 C-Atomen, einen Alkenylrest mit 1 bis 4 C-Atomen oder einen $-CO-R^4$- Rest bedeutet, wobei $R^4$ einen gegebenenfalls durch Halogen oder Alkoxy substituierten Alkylrest mit 1 bis 4 C-Atomen oder einen aromatischen Rest bedeutet und $R^3$ ferner einen $-CO-NH-R^5$- Rest bedeutet, wobei $R^5$ einen Alkylrest mit 1 bis 4 C-Atomen oder einen aromatischen Rest bedeutet, ausgezeichnet zur Regulierung des Pflanzenwachstums geeignet sind und eine sehr gute Pflanzenverträglichkeit zeigen.

Die neuen Verbindungen der Formel I können sterisch in der Z- oder in der E-Form vorliegen. In den meisten Fällen liegen vorwiegend Gemische vor.

Weiterhin wurde gefunden, dass man β-Triazolyloxime der Formel I erhält, wenn man β-Triazoloxime der Formel ($R^3$ = H)

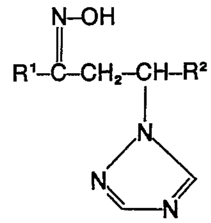

I

in welcher $R^1$ und $R^2$ die oben angegebene Bedeutung haben, mit Alkyl-, Alkenyl- und Benzylhalogeniden der Formel

$$R^3-X \qquad II$$

in der $R^3$ die oben angegebene Bedeutung hat und X Chlor oder Brom bedeutet oder mit Säurechloriden oder Säureanhydriden der Formel

$$R^4-COO-Y \qquad III$$

in der $R^4$ die oben angegebene Bedeutung hat und Y Chlor, Brom oder den Rest $-O-CO-R^4$ bedeutet, oder ferner mit einem Isocyanat der Formel

$$R^5-N=C=O \qquad IV$$

oder mit einem Carbamoylchlorid der Formel

$$R^5-NH-COCl \qquad V$$

in welcher $R^5$ die oben angegebene Bedeutung hat, gegebenenfalls in Gegenwart eines basischen Katalysators und gegebenenfalls in Gegenwart eines Verdünnungsmittels umsetzt.

Ferner wurde gefunden, dass man Verbindungen der Formel I erhält, wenn man β-Triazolylketone der Formel

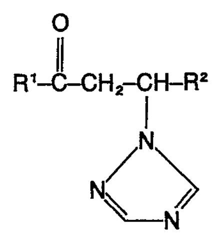

VI

in welcher $R^1$ und $R^2$ die oben angegebene Bedeutung haben, mit Hydroxylamin-Derivaten der Formel

$$H_2N-O-R^3 \qquad VII$$

in der $R^3$ Wasserstoff, Alkyl mit 1 bis 4 C-Atomen, Alkenyl mit 1 bis 4 C-Atomen oder Benzyl bedeutet, gegebenenfalls in Gegenwart eines basischen oder sauren Katalysators und gegebenenfalls in Gegenwart eines Verdünnungsmittels umsetzt. Dieses Verfahren wird bevorzugt.

In den Formeln bedeuten $R^1$ und $R^2$, die gleich oder verschieden sein können, beispielsweise

Methyl, Propyl, n-Butyl, sek.-Butyl, iso-Butyl, tert.-Butyl, n-Pentyl, Neopentyl, Hexyl, Furanyl, Thiophenyl, Naphthyl, Biphenylyl, Phenyl, m- und p-Tolyl, p-tert.-Butyl-phenyl, o- und p-Methoxyphenyl, 2,4-Dimethoxyphenyl, p-Fluorphenyl, o- und p-Chlorphenyl, p-Bromphenyl, 2,4-Dichlorphenyl, p-Nitrophenyl und p-Trifluormethyl-phenyl.

Die Verbindungen der Formel VI sind aus der Deutschen Offenlegungsschrift 2 634 511 bekannt, oder sie können nach den darin beschriebenen Verfahren leicht hergestellt werden.

Alkyl-, Alkenyl- und Benzylhalogenide der Formel II oder die Säurechloride und Säureanhydride der Formel III oder die Alkyl- und Arylisocyanate der Formel IV oder die Carbamoylchloride der Formel V oder die Hydroxylamine der Formel VII sind bekannt oder lassen sich nach üblichen Verfahren leicht herstellen.

Die Umsetzung von β-Triazolylketonen der Formel VI mit Ausgangsstoffen der Formel VII wird zweckmässig in Wasser oder in einem mit Wasser mischbaren Lösungsmittel durchgeführt. Hierzu gehören beispielsweise Alkohole, wie Methanol, Äthanol, Propanol, Äthylenglykol, 2-Methoxyäthanol; Äther wie Tetrahydrofuran und Dioxan; Amide, wie Dimethylformid, Diäthylformamid, Dimethylacetamid, ferner N-Methylpyrrolidon und Hexamethyl-phosphorsäure oder Säuren, wie Ameisensäure, Essigsäure und Propionsäure. Dabei ist es zweckmässig, eine Base wie beispielsweise Natriumhydroxid, Kaliumhydroxid, Bariumhydroxid, Natriumacetat, Amine wie Triäthylamin, Piperidin und N-Methylpiperidin oder eine Säure wie Salzsäure, Schwefelsäure, Phosphorsäure, Ameisensäure oder Essigsäure zuzusetzen.

Die Reaktionstemperaturen können in einem weiten Bereich variiert werden. Im allgemeinen arbeitet man zwischen 0 und 70 °C, vorzugsweise zwischen 0 und 40 °C.

Die Umsetzungen der β-Azolyloxime der Formel I ($R^3$ = H) mit Ausgangsstoffen der Formel II, III, IV und V werden zweckmässig ohne Verdünnungsmittel oder in einem inerten Lösungsmittel durchgeführt. Hierzu gehören beispielsweise Acetonitril; Äther, wie Diäthyl-, Dipropyl-, Dibutyläther, Tetrahydrofuran, Dioxan und Dimethoxyäthan; Ester wie Essigsäureäthylester; Ketone wie Aceton und Methyläthylketon; chlorierte Kohlenwasserstoffe wie Methylenchlorid, Chloroform, Tetrachlorkohlenstoff, Dichloräthan, Tetrachloräthan, Chlorbenzol; Kohlenwasserstoffe wie Benzol, Toluol und Xylol. Dabei ist es zweckmässig, eine Base wie beispielsweise Natriumcarbonat, Kaliumcarbonat, Calciumcarbonat, Natriumacetat; Amine wie Triäthylamin, Pyridin, Imidazol oder Piperidin als Hilfsbasen oder in katalytischen Mengen zuzusetzen. Die Reaktionstemperatur kann dabei zwischen –25 bis +100 °C, vorzugsweise zwischen +10 und +80 °C, variiert werden.

Die so erhaltenen Verbindungen der Formel I werden nach den üblichen Methoden isoliert und gegebenenfalls gereinigt.

Als Beispiele für besonders wirksame Vertreter der neuen Wirkstoffe seien folgende Verbindungen genannt:

4-(1′,2′,4′-Triazolyl-(1′))-5,5-dimethyl-hexan-2-on-oxim,

3-(1′,2′,4′-Triazolyl-(1′))-2,2-dimethyl-octan-5-on-oxim,

3-(1′,2′,4′-Triazolyl-(1′))-2,2-dimethyl-nonan-5-on-oxim,

3-(1′,2′,4′-Triazolyl-(1′))-2,2-dimethyl-decan-5-on-oxim,

3-(1′,2′,4′-Triazolyl-(1′))-2,2-dimethyl-undecan-5-on-oxim,

3-(1′,2′,4′-Triazolyl-(1′))-1-phenyl-4,4-dimethyl-pentan-1-on-oxim,

3-(1′,2′,4′-Triazolyl-(1′))-1-(4″-fluorphenyl)-4,4-dimethyl-pentan-1-on-oxim,

3-(1′,2′,4′-Triazolyl-(1′))-1-(4″-fluorphenyl)-4,4-dimethyl-pentan-1-on-O-methyl-oxim,

3-(1′,2′,4′-Triazolyl-(1′))-1-(4″-chlorphenyl)-4,4-dimethyl-pentan-1-on-oxim,

3-(1′,2′,4′-Triazolyl-(1′))-1-(4″-chlorphenyl)-4,4-dimethylpentan-1-on-O-acetyl-oxim,

3-(1′2′,4′-Triazolyl-(1′))-1-(4″-chlorphenyl)-4,4-dimethylpentan-1-on-O-propionyl-oxim,

3-(1′,2′,4′-Triazolyl-(1′))-1-(4″-bromphenyl)-4,4-dimethylpentan-1-on-oxim,

3-(1′,2′,4′-Triazolyl-(1′))-1-(4″-bromphenyl)-4,4-dimethylpentan-1-on-O-acetyl-oxim,

3-(1′,2′,4′-Triazolyl-(1′))-1-(4″-bromphenyl)-4,4-dimethylpentan-1-on-O-propionyloxim,

3-(1′,2′,4′-Triazolyl-(1′))-1-(2″,4″-dichlorphenyl)-4,4-dimethyl-pentan-1-on-oxim,

3-(1′,2′,4′-Triazolyl-(1′))-1-(4″-tolyl)-4,4-dimethyl-pentan-1-on-oxim,

3-(1′,2′,4′-Triazolyl-(1′))-1-(3″-tolyl)-4,4-dimethyl-pentan-1-on-oxim,

3-(1′,2′,4′-Triazolyl-(1′))-1-(4″-methoxyphenyl)-4,4-dimethyl-pentan-1-on-oxim,

1-(1′,2′,4′-Triazolyl-(1′))-1-(4″-chlorphenyl)-5,5-dimethylhexan-3-on-oxim,

3-(1′,2′,4′-Triazolyl-(1′))-1-(4″-biphenylyl)-4,4-dimethylpentan-1-on-oxim,

3-(1′,2′,4′-Triazolyl-(1′))-1-(2″-furfuryl)-4,4-dimethylpentan-1-on-oxim,

3-(1′,2′,4′-Triazolyl-(1′))-1-(2″-thiophenyl)-4,4-dimethylpentan-1-on-oxim,

3-(1′,2′,4′-Triazolyl-(1′))-1,3-diphenyl-propan-1-on-oxim,

3-(1′,2′,4′-Triazolyl-(1′))-1,3-diphenyl-propan-1-on-O-acetyloxim,

3-(1′,2′,4′-Triazolyl-(1′))-1,3-diphenyl-propan-1-on-O-(methyl-carbamoyl)-oxim,

3-(1′,2′,4′-Triazolyl-(1′))-1,3-diphenyl-propan-1-on-O-(p-chlorphenyl-carbamoyl)-oxim,

3-(1′,2′,4′-Triazolyl-(1′))-1-phenyl-3-(4″-tolyl)-propan-1-on-oxim,

3-(1′,2′,4′-Triazolyl-(1′))-1-phenyl-3-(4″-methoxyphenyl)-propan-1-on-oxim,

3-(1′,2′,4′-Triazolyl-(1′))-1-phenyl-3-(4″-tert.-butylphenyl)-propan-1-on-oxim,

3-(1′,2′,4′-Triazolyl-(1′))-1-phenyl-3-(4″-nitrophenyl)-propan-1-on-oxim,

3-(1′,2′,4′-Triazolyl-(1′))-1-phenyl-3-(1″-naphthyl)-propan-1-on-oxim,

3-(1′,2′,4′-Triazolyl-(1′))-1-(4″-trifluormethylphenyl)-3-phenyl-propan-1-on-oxim,

3-(1',2',4'-Triazolyl-(1'))-1-(4''-chlorphenyl)-3-(4''-tolyl)-propan-1-on-oxim,

3-(1',2',4'-Triazolyl-(1'))-1-(2''-methoxyphenyl)-3-(4''-chlorphenyl)-propan-1-on-oxim,

3-(1',2',4'-Triazolyl-(1'))-1-phenyl-4,4-dimethyl-pentan-1-on-O-methyl-oxim,

3-(1',2',4'-Triazolyl-(1'))-1-phenyl-4,4-dimethyl-pentan-1-on-O-allyloxim,

3-(1',2',4'-Triazolyl-(1'))-1-(4''-chlorphenyl)-4,4-pentan-1-on-O-methyloxim,

3-(1',2',4'-Triauolyl-(1'))-1-(4''-chlorphenyl)-4,4-pentan-1-on-O-allyloxim,

3-(1',2',4'-Triazolyl-(1'))-1-(4''-chlorphenyl)-4,4-pentan-1-on-O-benzoyl-oxim,

3-(1',2',4'-Triazolyl-(1'))-1-(4''-bromphenyl)-4,4-pentan-1-on-O-methyl-oxim,

3-(1',2',4'-Triazolyl-(1'))-1-(4''-bromphenyl)-4,4-pentan-1-on-O-allyl-oxim,

3-(1',2',4'-Triazolyl-(1'))-1-(4''-bromphenyl)-3,3-pentan-1-on-O-benzyl-oxim.

Die folgenden Beispiele erläutern die Herstellung der neuen Verbindungen.

Beispiel 1

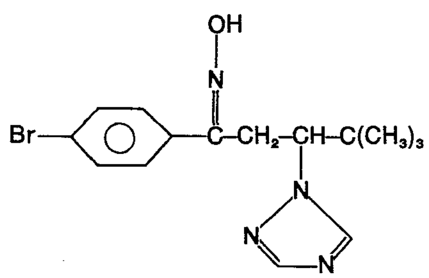

In eine Lösung von 33,6 g 3-(1',2',4'-Triazolyl-(1'))-1-(4''-bromphenyl)-4,4-dimethyl-pentan-1-on in 100 ml Äthanol werden unter gutem Rühren 9,7 g Hydroxylaminhydrochlorid eingetragen. Nach 24stündigem Rühren bei Raumtemperatur wird das Gemisch eingeengt. Der Rückstand wird nacheinander mit 250 ml Methylenchlorid und 200 ml einer 5%igen (Gewichtsprozent) wässrigen Natriumhydrogencarbonat-Lösung versetzt und 10 Minuten gerührt. Die organische Schicht wird über $Na_2SO_4$ getrocknet und im Vakuum eingeengt. Der verbleibende kristalline, farblose Rückstand wird mit 100 ml Petroläther gewaschen und abgesaugt. Man erhält 30,2 g (86,1% d.Th.) 3-(1',2',4'-Triazolyl-(1'))-1-(4''-bromphenyl)-4,4-dimethyl-pentan-1-on-oxim vom Schmelzpunkt 185 bis 186 °C. (Wirkstoff Nr. 1)

Beispiel 2

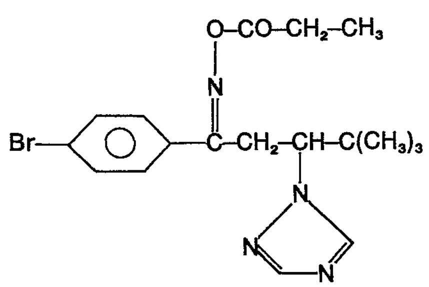

17 g 3-(1',2',4'-Triazolyl-(2'))-1-(4''-bromphenyl)-4,4dimethyl-pentan-1-on-oxim und 0,5 g Imidazol werden in 60 g Propionsäureanhydrid aufgelöst und 6 Stunden bei 45 °C gerührt. Danach wird das Lösungsmittel im Vakuum abdestilliert, der Rückstand nacheinander mit 200 ml Methylenchlorid und 150 ml einer 5%igen Natrium hydrogencarbonat-Lösung versetzt und ½ Stunde gerührt. Die organische Phase wird danach zweimal mit je 100 ml Wasser gewaschen, über $Na_2SO_4$ getrocknet, abfiltriert und schliesslich eingeengt. Der Rückstand wird mit 100 ml Petroläther gewaschen, abgesaugt und getrocknet.

Man erhält 16,3 g (83% d.Th.) 3-(1',2',4'-Triazolyl-(1'))-1-(4''-bromphenyl)-4,4-Dimethyl-pentan-1-on-O-propionyloxim als weisse, analysenreine Kristalle vom Schmelzpunkt 106 bis 108 °C. (Wirkstoff Nr. 2)

Beispiel 3

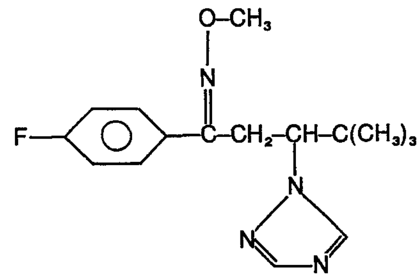

In eine Lösung von 28,7 g 3-(1',2',4'-Triazolyl-(1'))-1-(4''-fluorphenyl)-4,4-dimethyl-pentan-1-on in 100 ml Methanol werden unter gutem Rühren 11,6 g O-Methylhydroxylamin-hydrochlorid und 14,1 g Triäthylamin eingetragen. Nach 2stündigem Rühren bei 50 °C wird das Gemisch eingeengt. Der Rückstand wird mit 250 ml Methylenchlorid und 200 ml Wasser ausgeschüttelt. Die organische Phase wird über Natriumsulfat getrocknet und im Vakuum eingeengt. Der verbleibende feste, farblose Rückstand wird mit 80 ml Diäthyläther bei −5 °C zwei Stunden digeriert, abgesaugt, mit wenig kaltem Äther gewaschen und getrocknet.

Man erhält 14 g (46% d.Th.) 3-(1',2',4'-Triazolyl-(1'))-1-(4''-fluorphenyl)-4,4-dimethyl-pentan-1-on-O-methyloxim als weisse Kristalle vom Schmelzpunkt 109 bis 111 °C. (Wirkstoff Nr. 3)

Beispiel 4

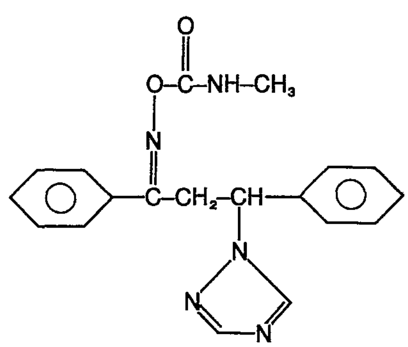

In eine Lösung von 29,2 g 3-(1',2',4'-Triazolyl-(1'))-1,3-diphenyl-propan-1-on-oxim und 0,5 g Triäthylamin in 150 ml trockenem Dioxan werden 8,5 g Methylisocyanat unter gutem Rühren bei Raumtemperatur in 10 Minuten zugetropft.

Nach 8stündigem Rühren bei 40 °C wird das Gemisch eingeengt. Der feste, farblose Rückstand wird mit 50 ml Diäthyläther bei 0 °C gerührt, abgesaugt und getrocknet. Man erhält 28,3 g (81% d.Th.) 3-(1',2',4'-Triazolyl-(1'))-1,3-diphenyl-propan-1-on-O-(methylcarbamoyl)-oxim als weisse Kristalle vom Schmelzpunkt 105 bis 108 °C. (Wirkstoff Nr. 4)

In entsprechender Weise können die in der folgenden Tabelle 1 aufgeführten Verbindungen hergestellt werden.

Tabelle

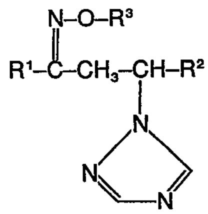

| Wirkstoff Nr. | $R^1$ | $R^2$ | $R^3$ | Schmelzpunkt °C |
|---|---|---|---|---|
| 5 | $CH_3-(CH_3)_2-$ | $-C(CH_3)_3$ | H | 141–143 |
| 6 | $CH_3-(CH_2)_3-$ | $-CH(CH_3)_2$ | H | 73– 76 |
| 7 | $CH_3-(CH_2)_3-$ | $-C(CH_3)_3$ | H | 152–154 |
| 8 | $CH_3-(CH_2)_4-$ | $-C(CH_3)_3$ | H E-Form<br>Z-Form | 141–143<br>81– 83 |
| 9 | $C_6H_5-$ | $-C(CH_3)_3$ | H | 168–170 |
| 10 | 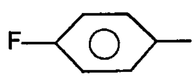 | $-C(CH_3)_3$ | H | 167–169 |
| 11 | 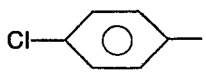 | $-C(CH_3)_3$ | H | 170–172 |
| 12 | $(CH_3)_3C-CH_2-$ | 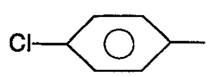 | H | 136–138 |
| 13 | 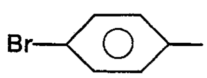 | $-C(CH_3)_3$ | $CH_3-CO-$ | 128–130 |
| 14 | 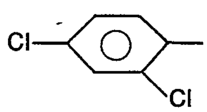 | $-C(CH_3)_3$ | H | 202–205 |
| 15 | 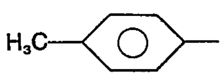 | $-C(CH_3)_3$ | H | 178–180 |
| 16 | 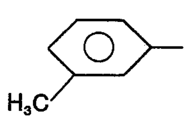 | $-C(CH_3)_3$ | H | 149–151 |
| 17 | 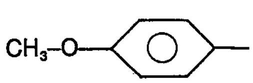 | $-C(CH_3)_3$ | H | 174–176 |
| 18 | 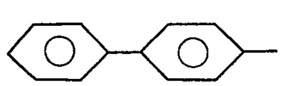 | $-C(CH_3)_3$ | H | 180–183 |
| 19 | 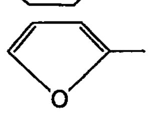 | $-C(CH_3)_3$ | H | 159–160 |
| 20 | 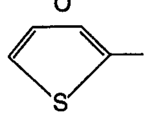 | $-C(CH_3)_3$ | H | 139–142 |

Tabelle (Fortsetzung)

| Wirkstoff Nr. | $R^1$ | $R^2$ | $R^3$ | Schmelzpunkt °C |
|---|---|---|---|---|
| 21 | $C_6H_5$– | $C_6H_5$ | H | 153–156 |
| 22 | $C_6H_5$– | $C_6H_5$– | $CH_3$–CO– | 100–101 |
| 23 | $C_6H_5$– | 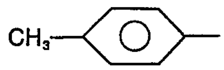 | H | 181–185 |
| 24 | $C_6H_5$– | 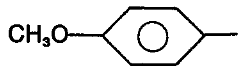 | H | 132–134 |
| 25 | 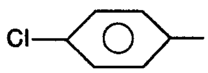 | 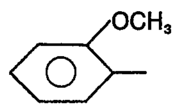 | H | 185–186 |
| 26 | $C_6H_5$– | 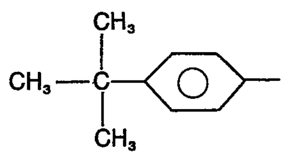 | H | 149–151 |
| 27 | $C_6H_5$– | 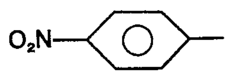 | H | 194–196 |
| 28 | $C_6H_5$– | 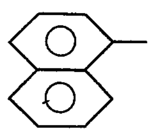 | H | 173–175 |
| 29 | 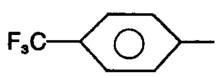 | $C_6H_5$ | H | 206–208 |
| 30 | 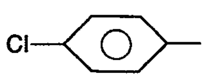 | 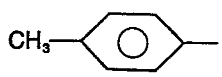 | H | 211–213 |
| 31 | $C_6H_5$– | $(CH_3)_3C$– | $CH_3$–CO– | 131–133 |
| 32 | $C_6H_5$– | $(CH_3)_3C$– | $CH_3CH_2CO$– | 60– 62 |
| 33 | $C_6H_5$ | $(CH_3)_3C$– | $C_6H_5$–CO– | 137–139 |
| 34 | 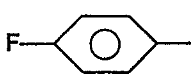 | $(CH_3)_3C$– | $CH_3$–CO– | 132–134 |
| 35 | 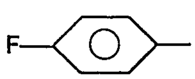 | $(CH_3)_3C$– | $CH_3CH_2CO$– | 104–106 |
| 36 | 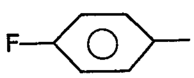 | $(CH_3)_3C$– | $C_6H_5$–CO– | 155–157 |
| 37 | 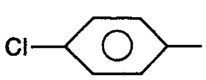 | $(CH_3)_3C$– | $CH_3$–CO– | 123–126 |
| 38 | 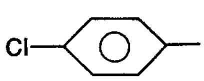 | $(CH_3)_3C$– | $CH_3CH_2CO$– | 102–104 |
| 39 | 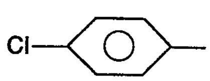 | $(CH_3)_3C$– | $(CH_3)_2CHCH_2CO$– | 98–100 |
| 40 | $(CH_3)_3C$–$CH_2$– | 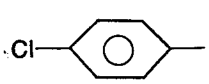 | $CH_3CO$– | 103–110 |

Tabelle (Fortsetzung)

| Wirkstoff Nr. | $R^1$ | $R^2$ | $R^3$ | Schmelzpunkt °C |
|---|---|---|---|---|
| 41 | 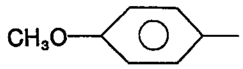 | $(CH_3)_3C-$ | $CH_3-CO-$ | 119–121 |
| 42 | 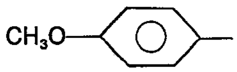 | $(CH_3)_3C-$ | $CH_3CH_2CO-$ | 99–101 |
| 43 | 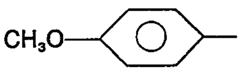 | $(CH_3)_3C-$ | $C_6H_5-CO-$ | 121–123 |
| 44 | 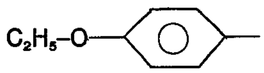 | $(CH_3)_3C-$ | H | 165–168 |
| 45 | 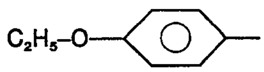 | $(CH_3)_3C-$ | $CH_3-CO-$ | 111–113 |
| 46 | 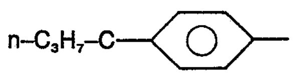 | $(CH_3)_3C-$ | H | 150–152 |
| 47 | 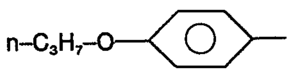 | $(CH_3)_3C-$ | $CH_3-CO-$ | 97– 99 |
| 48 | 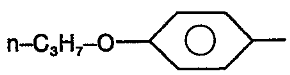 | $(CH_3)_3C-$ | $CH_3CH_2CO-$ | 81– 83 |
| 49 | 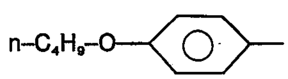 | $(CH_3)_3C-$ | H | 149–151 |
| 50 | 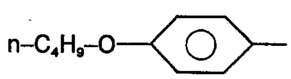 | $(CH_3)_3C-$ | $CH_3CO-$ | 119–121 |
| 51 | 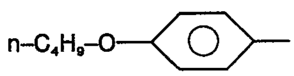 | $(CH_3)_3C-$ | $CH_3CH_3CO-$ | 70– 71 |
| 52 | $C_6H_5-$ | 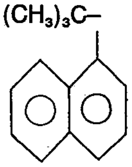 | $CH_3-CO-$ | Harz |
| 53 | $C_6H_5$ | 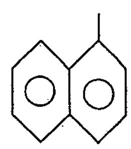 | $CH_3CH_2CO-$ | Harz |

Die neuen Verbindungen können praktisch alle Entwicklungsstadien einer Pflanze verschiedenartig beeinflussen und werden deshalb als Pflanzenwachstumsregulatoren eingesetzt. Die Wirkungsvielfalt der Pflanzenwachstumsregulatoren hängt ab vor allem

a) von der Pflanzenart und -sorte,

b) von dem Zeitpunkt der Applikation, bezogen auf das Entwicklungsstadium der Pflanze, und von der Jahreszeit,

c) von dem Applikationsort und -verfahren (Samenbeize, Bodenbehandlung oder Blattapplikation),

d) von den geoklimatischen Faktoren, z.B. Sonnenscheindauer, Durchschnittstemperatur, Niederschlagsmenge,

e) von der Bodenbeschaffenheit (einschliesslich Düngung),

f) von der Formulierung bzw. Anwendungsform des Wirkstoffs und schliesslich

g) von den angewendeten Konzentrationen der aktiven Substanz.

Aus der Reihe der verschiedenartigen Anwendungsmöglichkeiten der Pflanzenwachstumsregulatoren im Pflanzenanbau, in der Landwirtschaft und im Gartenbau werden einige nachstehend erwähnt.

A. Mit den erfindungsgemäss verwendbaren Verbindungen lässt sich das vegetative Wachstum der Pflanzen stark hemmen, was sich insbesondere in einer Reduzierung des Längenwachstums äussert. Die behandelten Pflanzen weisen demgemäss einen gedrungenen Wuchs auf; ausserdem ist eine dunklere Blattfärbung zu beobachten.

Als vorteilhaft für die Praxis erweist sich z.B. die Verringerung des Grasbewuchses an Strassenrändern, Kanalböschungen und auf Rasenflächen wie Park-, Sport- und Obstanlagen, Zierrasen und Flugplätzen, so dass der arbeits- und kostenaufwendige Rasenschnitt reduziert werden kann.

Von wirtschaftlichem Interesse ist auch die Erhöhung der Standfestigkeit von lageranfälligen Kulturen wie Getreide, Mais und Soja. Die dabei verursachte Halmverkürzung und Halmverstärkung verringern oder beseitigen die Gefahr des «Lagerns» (des Umknickens) von Pflanzen unter ungünstigen Witterungsbedingungen vor der Ernte.

Wichtig ist auch die Anwendung von Wachstumsregulatoren zur Hemmung des Längenwachstums und zur zeitlichen Veränderung des Reifeverlaufs bei Baumwolle. Damit wird ein vollständig mechanisiertes Beernten dieser wichtigen Kulturpflanze ermöglicht.

Durch Anwendung von Wachstumsregulatoren kann auch die seitliche Verzweigung der Pflanzen vermehrt oder gehemmt werden. Daran besteht Interesse, wenn z.B. bei Tabakpflanzen die Ausbildung von Seitentrieben (Geiztrieben) zugunsten des Blattwachstums gehemmt werden soll.

Mit Wachstummshemmern lässt sich beispielsweise bei Winterraps auch die Frostresistenz erheblich erhöhen. Dabei werden einerseits das Längenwachstum und die Entwicklung einer zu üppigen (und dadurch besonders frostanfälligen) Blatt- bzw. Pflanzenmasse gehemmt. Anderseits werden die jungen Rapspflanzen nach der Aussaat und vor dem Einsetzen der Winterfröste trotz günstigen Wachstumsbedingungen im vegetativen Entwicklungsstadium zurückgehalten. Dadurch wird auch die Frostgefährdung solcher Pflanzen beseitigt, die zum vorzeitigen Abbau der Blühhemmung und zum Übergang in die generative Phase neigen. Auch bei anderen Kulturen, z.B. Wintergetreide, ist es vorteilhaft, wenn die Bestände durch Behandlung mit erfindungsgemässen Verbindungen im Herbst zwar gut bestockt, aber nicht zu üppig in den Winter hineingehen. Dadurch kann der erhöhten Frostempfindlichkeit und – wegen der relativ geringen Blatt- bzw. Pflanzenmasse – dem Befall mit verschiedenen, insbesondere pilzlichen Krankheiten vorgebeugt werden. Die Hemmung des vegetativen Wachstums ermöglicht ausserdem bei vielen Kulturpflanzen eine dichtere Bepflanzung, so dass ein Mehrertrag bezogen auf die Bodenfläche erzielt werden kann.

B. Mit den neuen Wirkstoffen lassen sich Mehrerträge sowohl an Pflanzenteilen als auch an Pflanzeninhaltsstoffen erzielen. So ist es beispielsweise möglich, das Wachstum grösserer Mengen an Knospen, Blüten, Blättern, Früchten, Samenkörnern, Wurzeln und Knollen zu induzieren, den Gehalt an Zucker in Zuckerrüben, Zuckerrohr sowie Zitrusfrüchten zu erhöhen, den Proteingehalt in Getreide oder Soja zu steigern oder Gummibäume zum vermehrten Latexfluss zu stimulieren.

Dabei können die neuen Stoffe Ertragssteigerungen durch Eingriffe in den pflanzlichen Stoffwechsel bzw. durch Förderung oder Hemmung des vegetativen und/oder des generativen Wachstums verursachen.

C. Mit Pflanzenwachstumsregulatoren lassen sich schliesslich sowohl eine Verkürzung bzw. Verlängerung der Wachstumsstadien als auch eine Beschleunigung bzw. Verzögerung der Reife der geernteten Pflanzenteile vor oder nach der Ernte erreichen.

Von wirtschaftlichem Interesse ist beispielsweise die Ernteerleichterung, die durch das zeitlich konzentrierte Abfallen oder Vermindern der Haftfestigkeit am Baum bei Zitrusfürchten, Oliven oder bei anderen Arten und Sorten von Kern-, Stein- und Schalenobst ermöglicht wird. Derselbe Mechanismus, das heisst die Förderung der Ausbildung von Trenngewebe zwischen Frucht- bzw. Blatt und Sprossteil der Pflanze ist auch für ein gut kontrollierbares Entblättern der Bäume wesentlich.

Die Wirkung der neuen Verbindungen ist besser als bei bekannten Wachstumregulatoren. Die Wirkung zeigt sich sowohl bei Monokotylen, z.B. Getreide wie Weizen, Gerste, Roggen, Hafer und Reis oder Mais, als auch bei Dikotylen (z.B. Sonnenblumen, Tomaten, Soja, Reben, Baumwolle und vor allem Raps) und verschiedenen Zierpflanzen wie Chrysanthemen, Poinsettien und Hibiskus.

Die neuen β-Triazolyloxime können den Kulturpflanzen sowohl vom Samen her (als Saatgutbeizmittel), als auch über den Boden, d.h. durch die Wurzel sowie durch Spritzung über das Blatt zugeführt werden.

Infolge der hohen Pflanzenverträglichkeit kann die Aufwandmenge stark variiert werden.

Bei der Saatgutbehandlung werden im allgemeinen Wirkstoffmengen von 0,001 bis 50 g je Kilogramm Saatgut, vorzugsweise 0,01 bis 10 g benötigt.

Für die Blatt- und Bodenbehandlung sind im allgemeinen Gaben von 0,01 bis 12 kg/ha, bevorzugt 0,25 bis 3 kg/ha als ausreichend zu betrachten.

Die erfindungsgemässen Mittel können in Form üblicher Formulierungen angewendet werden, wie Lösungen, Emulsionen, Suspensionen, Stäube, Pulver, Pasten und Granulate. Die Anwendungsformen richten sich ganz nach den Verwendungszwecken; sie sollen in jedem Fall eine feine und gleichmässige Verteilung der wirksamen Substanz gewährleisten. Die Formulierungen werden in bekannter Weise hergestellt, z.B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel verwendet werden können. Als

Hilfsstoffe kommen dafür im wesentlichen in Frage: Lösungsmittel wie Aromaten (z.B. Xylol, Benzol), chlorierte Aromaten (z.B. Chlorbenzole), Paraffine (z.B. Erdölfraktionen), Alkohole (z.B. Methanol, Butanol), Amine (z.B. Äthanolamin), Dimethylformamid und Wasser; feste Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoxyäthylen-Fettalkohol-Äther, Alkylsulfonate) und Dispergiermittel, wie Lignin, Sulfitablaugen und Methylcellulose.

Die Formulierungen enthalten im allgemeinen zwischen 0,1 und 95 Gewichtsprozent Wirkstoff, vorzugsweise zwischen 0,5 und 90.

Die Formulierungen, bzw. die daraus hergestellten gebrauchsfertigen Zubereitungen, wie Lösungen, Emulsionen, Suspensionen, Pulver, Stäube, Pasten oder Granulate, werden in bekannter Weise angewendet, beispielsweise im Vorauflaufverfahren, im Nachauflaufverfahren oder als Beizmittel.

Die erfindungsgemässen Mittel können in diesen Aufwendungsformen auch zusammen mit anderen Wirkstoffen vorliegen, wie z.B. Herbiziden, Insektiziden, Wachstumsregulatoren und Fungiziden, oder auch mit Düngemitteln vermischt und ausgebracht werden. Beim Vermischen mit Wachstumsregulatoren erhält man dabei in vielen Fällen eine Vergrösserung des Wirkungsspektrums. Bei einer Anzahl solcher Wachstumsregulatormischungen treten auch synergistische Effekte auf, d.h. die Wirksamkeit des Kombinationsproduktes ist grösser als die addierten Wirksamkeiten der Einzelkomponenten.

In den nachfolgenden Beispielen wird die Wirkung der erfindungsgemäss verwendbaren Stoffe als Pflanzenwachstumsregulatoren dargestellt, ohne die Möglichkeit weiterer Anwendungen als Wachstumsregulatoren auszuschliessen.

Die folgenden Beispiele erläutern die biologische Wirkung der neuen Verbindungen.

I. Beispiele aus Gewächshausversuchen

Zu Gewächshausversuchen wurden die Testpflanzen in Kunststoffschalen von 12,5 cm Durchmesser auf ausreichend mit Nährstoffen versorgten Torfkultursubstraten herangezogen. Im Vorauflaufverfahren wurden die Prüfsubstanzen in verschiedenen Konzentrationen als wässrige Aufbereitungen am Tage der Aussaat auf das Saatbett gegossen. Bei der Blattbehandlung wurden die Pflanzen bei einer Wuchshöhe von etwa 10 cm mit wässrigen Aufbereitungen der Prüfsubstanzen in unterschiedlichen Konzentrationen besprüht. Die wachstumsregulierende Wirkung der geprüften Substanzen wurde durch Wuchshöhenmessung zu Versuchsende belegt und die so gewonnenen Messwerte zu der Wuchshöhe der unbehandelten Pflanzen in Relation gesetzt. Als Vergleichssubstanzen dienten die bekannten Wirkstoffe CCC (A) und 1-(4′-Bromphenyl)-1-allyloxy-2-(1″,2″,4″-triazolyl-(1″))-äthan (B).

Die Einzelwerte sind den folgenden Tabellen zu entnehmen.

Vergleichssubstanzen:

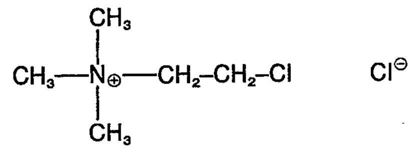

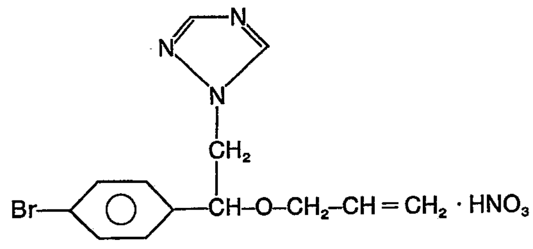

II. Beispiele aus Vegetationsversuchen

In Vegetationsversuchen wurde Sommerraps der Sorte «Kosa» auf einem neutralen, lehmigen Sandboden, der ausreichend mit Nährstoffen versorgt wurde, in Metall-Gefässen angebaut.

Bei der Bodenbehandlung wurden die zu prüfenden Substanzen in wässrigen Aufbereitungen am Tage der Einsaat auf das Saatbett gespritzt. Die Blattbehandlung erfolgte bei einer Wuchshöhe von ca. 16 cm durch Besprühen der Pflanzen mit wässrigen Aufbereitungen der Testsubstanzen.

Die Aufwandmengen waren 5 und 10 mg Wirkstoff pro Gefäss. Jeweils 4 Gefässe bildeten eine Versuchsvariante. Bei Versuchsende (Reife) wurden die Wuchshöhen gemessen und zu unbehandelten Pflanzen in Relation gesetzt.

III. Beispiel aus einem Baumwollversuch in Wasserkultur

Baumwollpflanzen der Sorte «Delta Pine», die auf Quarzsand angezogen waren, wurden in 4 Liter fassenden Behältern auf Wasserkultur gesetzt und ausreichend mit Nährlösung und Spurenelementen versorgt. Die Wirkstoffzugabe in die Nährlösung erfolgte bei einer Pflanzenhöhe von ca. 13 cm. Die Pflanzen blieben in Dauerkontakt mit den Wirkstoffen. Nach 46 Tagen wurden die Wuchshöhen gemessen und zu unbehandelten Pflanzen in Relation gesetzt.

Beispiel I.1

Einfluss auf das Längenwachstum von Sommergerste der Sorte «Union»

Vorauflaufverfahren; Versuchsdauer: 19 Tage

| Wirkstoff Nr. | konz. mg/Gefäss | Wuchshöhen cm | % |
|---|---|---|---|
| Kontrolle (unbehandelt) | – | 26,3 | 100 |
| A | 3 | 25,0 | 95,1 |
| | 12 | 22,0 | 83,7 |
| B | 3 | 25,0 | 95,1 |
| | 12 | 22,0 | 83,7 |
| 1 | 3 | 24,0 | 91,3 |
| | 12 | 21,0 | 79,9 |

Beispiel I.2
Einfluss auf das Längenwachstum von Sommerraps der Sorte «Petronova»
Vorauflaufverfahren; Versuchsdauer: 15 Tage

| Wirkstoff Nr. | konz. mg/Gefäss | Wuchshöhen cm | % |
|---|---|---|---|
| Kontrolle (unbehandelt) | – | 12,1 | 100 |
| A | 3 | 12,0 | 99,2 |
| | 12 | 12,0 | 99,2 |
| B | 3 | 11,5 | 95,0 |
| | 12 | 10,0 | 82,6 |
| 1 | 3 | 6,0 | 49,6 |
| | 12 | 4,6 | 37,2 |

Beispiel I.3
Einfluss auf das Längenwachstum von Sommerraps der Sorte «Petronova»
Blattbehandlung; Versuchsdauer: 22 Tage

| Wirkstoff Nr. | konz. mg/Gefäss | Wuchshöhen cm | % |
|---|---|---|---|
| Kontrolle (unbehandelt) | – | 18,6 | 100 |
| A | 1,5 | 17,5 | 94,1 |
| | 6 | 17,5 | 94,1 |
| B | 1,5 | 17,5 | 94,1 |
| | 6 | 17,0 | 91,4 |
| 1 | 1,5 | 16,5 | 88,7 |
| | 6 | 14,5 | 78,0 |

Beispiel I.4
Einfluss auf das Längenwachstum von Sommerraps der Sorte «Petronova»
Vorauflaufverfahren; Versuchsdauer: 16 Tage

| Wirkstoff Nr. | konz. mg/Gefäss | Wuchshöhen cm | % |
|---|---|---|---|
| Kontrolle (unbehandelt) | – | 13,5 | 100 |
| A | 3 | 13,0 | 96,3 |
| | 12 | 12,5 | 92,6 |
| B | 3 | 12,5 | 92,6 |
| | 12 | 11,0 | 81,5 |
| 11 | 3 | 9,0 | 66,7 |
| | 12 | 6,5 | 48,2 |
| 15 | 3 | 10,0 | 74,1 |
| | 12 | 8,0 | 59,3 |

Beispiel I.5
Einfluss auf das Längenwachstum von Sommerraps der Sorte «Petronova»
Vorauflaufverfahren; Versuchsdauer: 16 Tage

| Wirkstoff Nr. | konz. mg/Gefäss | Wuchshöhen cm | % |
|---|---|---|---|
| Kontrolle (unbehandelt) | – | 16,6 | 100 |
| A | 3 | 15,5 | 90,4 |
| | 12 | 14,5 | 87,4 |

Beispiel I.5 Fortsetzung

| Wirkstoff Nr. | konz. mg/Gefäss | Wuchshöhen cm | % |
|---|---|---|---|
| B | 3 | 15,0 | 90,4 |
| | 12 | 11,0 | 66,3 |
| 29 | 3 | 9,5 | 57,2 |
| | 12 | 8,5 | 51,2 |

Beispiel I.6
Einfluss auf das Längenwachstum von Soja der Sorte SRF 450
Blattbehandlung; Versuchsdauer: 40 Tage

| Wirkstoff Nr. | konz. mg/Gefäss | Wuchshöhen cm | % |
|---|---|---|---|
| Kontrolle (unbehandelt) | – | 28,9 | 100 |
| A | 1,5 | 28,0 | 96,9 |
| | 6 | 28,0 | 96,9 |
| 3 | 1,5 | 26,0 | 90,0 |
| | 6 | 24,0 | 83,0 |
| 11 | 1,5 | 25,0 | 86,5 |
| | 6 | 17,0 | 58,8 |
| 14 | 1,5 | 27,0 | 93,4 |
| | 6 | 24,0 | 83,0 |
| 15 | 1,5 | 24,0 | 83,0 |
| | 6 | 17,0 | 58,8 |
| 20 | 1,5 | 25,0 | 86,5 |
| | 6 | 24,0 | 83,0 |

Beispiel I.7
Einfluss auf das Längenwachstum von Sommerweizen der Sorte «Kolibri»
Vorauflaufverfahren; Versuchsdauer: 14 Tage

| Wirkstoff Nr. | konz. mg/Gefäss | Wuchshöhen cm | % |
|---|---|---|---|
| Kontrolle (unbehandelt) | – | 30,6 | 100 |
| A | 3 | 21,6 | 70,3 |
| | 12 | 20,0 | 65,4 |
| 10 | 3 | 20,0 | 65,4 |
| | 12 | 16,0 | 52,3 |

Beispiel I.8
Einfluss auf das Längenwachstum von Sommerraps der Sorte «Kosa»
Vorauflaufverfahren; Versuchsdauer: 16 Tage

| Wirkstoff Nr. | konz. mg/Gefäss | Wuchshöhen cm | % |
|---|---|---|---|
| Kontrolle (unbehandelt) | – | 15,3 | 100 |
| A | 3 | 14,0 | 91,5 |
| | 12 | 14,0 | 91,5 |
| 10 | 3 | 13,0 | 85,0 |
| | 12 | 9,5 | 62,1 |

Beispiel I.9
Einfluss auf das Längenwachstum von Sommerraps der Sorte «Kosa»
Vorauflaufverfahren; Versuchsdauer: 16 Tage

| Wirkstoff Nr. | konz. mg/Gefäss | Wuchshöhen cm | % |
|---|---|---|---|
| Kontrolle (unbehandelt) | – | 15,9 | 100 |
| A | 3 | 15,9 | 100 |
| | 12 | 14,5 | 91,2 |
| 17 | 3 | 11,5 | 72,3 |
| | 12 | 7,5 | 47,2 |

Beispiel I.10
Einfluss auf das Längenwachstum von Sommerraps der Sorte «Kosa»
Blattbehandlung; Versuchsdauer: 18 Tage

| Wirkstoff Nr. | konz. mg/Gefäss | Wuchshöhen cm | % |
|---|---|---|---|
| Kontrolle (unbehandelt) | – | 16,4 | 100 |
| A | 1,5 | 15,5 | 94,5 |
| | 6 | 15,0 | 91,5 |
| 10 | 1,5 | 15,5 | 94,5 |
| | 6 | 13,5 | 82,3 |

Beispiel I.11
Einfluss auf das Längenwachstum von Sommerraps der Sorte «Kosa»
Blattbehandlung; Versuchsdauer: 18 Tage

| Wirkstoff Nr. | konz. mg/Gefäss | Wuchshöhen cm | % |
|---|---|---|---|
| Kontrolle (unbehandelt) | – | 16,8 | 100 |
| A | 1,5 | 15,0 | 89,3 |
| | 6 | 15,0 | 89,3 |
| 17 | 1,5 | 14,0 | 83,3 |
| | 6 | 12,0 | 71,4 |

Beispiel II.1
Einfluss auf das Längenwachstum von Sommerraps der Sorte «Kosa»
Vorauflaufverfahren

| Wirkstoff Nr. | konz. mg/Gefäss | Wuchshöhen cm | % |
|---|---|---|---|
| Kontrolle (unbehandelt) | – | 108,7 | 100 |
| B | 5 | 99,7 | 91,7 |
| | 10 | 93,3 | 85,8 |
| 1 | 5 | 68,5 | 63,0 |
| | 10 | 54,0 | 49,7 |
| 11 | 5 | 72,9 | 67,1 |
| | 10 | 60,6 | 55,8 |
| 15 | 5 | 98,0 | 90,2 |
| | 10 | 91,0 | 83,7 |

Beispiel II.2
Einfluss auf das Längenwachstum von Sommerraps der Sorte «Korsa»
Blattbehandlung

| Wirkstoff Nr. | konz. mg/Gefäss | Wuchshöhen cm | % |
|---|---|---|---|
| Kontrolle (unbehandelt) | – | 106,3 | 100 |
| B | 5 | 102,0 | 96,0 |
| | 10 | 97,0 | 91,3 |
| 1 | 5 | 85,5 | 80,4 |
| | 10 | 75,0 | 70,6 |
| 11 | 5 | 89,0 | 83,7 |
| | 10 | 77,0 | 72,4 |
| 15 | 5 | 94,0 | 88,4 |
| | 10 | 86,0 | 80,9 |

Beispiel III.1
Einfluss auf das Längenwachstum von Baumwolle der Sorte «Delta Pine» (Wasserkultur)

| Wirkstoff Nr. | konz. mg/Gefäss | Wuchshöhen cm | % |
|---|---|---|---|
| Kontrolle (unbehandelt) | – | 34,5 | 100 |
| 1 | 0,5 | 24,7 | 71,6 |
| | 1,5 | 17,7 | 51,3 |
| | 3,0 | 15,0 | 43,5 |

Beispiel 5

Man vermischt 90 Gewichtsteile der Verbindung 1 mit 10 Gewichtsteilen N-Methyl-α-pyrrolidon und erhält eine Lösung, die zur Anwendung in Form kleinster Tropfen geeignet ist.

Beispiel 6

20 Gewichtsteile der Verbindung 2 werden in einer Mischung gelöst, die aus 80 Gewichtsteilen Xylol, 10 Gewichtsteilen des Anlagerungsproduktes von 8 bis 10 Mol Äthylenoxid an 1 Mol Ölsäure N-monoäthanolamid, 5 Gewichtsteilen Calciumsalz der Dodecylbenzolsulfonsäure und 5 Gewichtsteilen des Anlagerungsproduktes von 40 Mol Äthylenoxid an 1 Mol Ricinusöl besteht. Durch Ausgiessen und feines Verteilen der Lösung in 100 000 Gewichtsteilen Wasser erhält man eine wässrige Dispersion, die 0,02 Gewichtsprozent des Wirkstoffs enthält.

Beispiel 7

20 Gewichtsteile der Verbindung 3 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Äthylenoxid an 1 Mol Isooctylphenol und 10 Gewichtsteilen des Anlagerungsproduktes von 40 Mol Äthylenoxid an 1 Mol Ricinusöl besteht. Durch Eingiessen und feines Verteilen der Lösung in 100 000 Gewichtsteilen Wasser erhält man eine wässrige Dispersion, die 0,02 Gewichtsprozent des Wirkstoffs enthält.

Beispiel 8

20 Gewichtsteile der Verbindung 1 werden in einer Mischung gelöst, die aus 25 Gewichtsteilen Cyclohexanol, 65 Gewichtsteilen einer Mineralölfraktion vom Siedepunkt 210 bis 280°C und 10 Gewichtsteilen des Anlagerungsproduktes von 40 Mol Äthylenoxid an 1 Mol Ricinusöl besteht. Durch Eingiessen und feines Verteilen der Lösung in 100 000 Gewichtsteilen Wasser erhält man eine wässrige Dispersion, die 0,02 Gewichtsprozent des Wirkstoffs enthält.

Beispiel 9

20 Gewichtsteile des Wirkstoffs 2 werden mit 3 Gewichtsteilen des Natriumsalzes der Diisobutylnaphthalin-α-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gewichtsprozent des Wirkstoffs enthält.

Beispiel 10

3 Gewichtsteile der Verbindung 3 werden mit 97 Gewichtsteilen feinteiligem Kaolin innig vermischt. Man erhält auf diese Weise ein Stäubemittel, das 3 Gewichtsprozent des Wirkstoffs enthält.

Beispiel 11

30 Gewichtsteile der Verbindung 4 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

Beispiel 12

40 Gewichtsteile des Wirkstoffs 1 werden mit 10 Teilen Natriumsalz eines Phenolsulfonsäure-harnstoff-formaldehyd-Kondensats, 2 Teilen Kieselgel und 48 Teilen Wasser innig vermischt. Man erhält eine stabile wässrige Dispersion. Durch Verdünnen mit 100 000 Gewichtsteilen Wasser erhält man eine wässrige Dispersion, die 0,04 Gewichtsprozent Wirkstoff enthält.

Beispiel 13

20 Teile des Wirkstoffs 2 werden mit 2 Teilen Calciumsalz der Dodecylbenzolsulfonsäure, 8 Teilen Fettalkohol-polyglykoläther, 2 Teilen Natriumsalz eines Phenolsulfonsäure-harnstoff-formaldehyd-Kondensats und 68 Teilen eines paraffinischen Mineralöls innig vermischt. Man erhält eine stabile ölige Dispersion.

**Patentansprüche für die Vertragsstaaten: BE, CH, DE, FR, GB, IT, LU, NL, SE**

1. β-Triazolyloxime der allgemeinen Formel

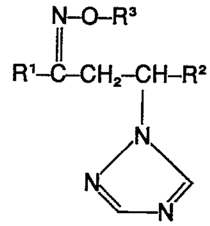

I

in der

$R^1$ und $R^2$ verschieden oder gleich sind und Alkyl mit 1 bis 6 C-Atomen, Furanyl, Thiophenyl, Naphthyl, Biphenylyl oder Phenyl bedeuten, wobei der Phenylrest durch Fluor, Chlor, Brom, Alkyl mit 1 bis 4 C-Atomen, Alkoxy mit 1 bis 4 C-Atomen, Trifluormethyl oder Nitro substituiert sein kann und

$R^3$ ein Wasserstoffatom, einen Alkylrest mit 1 bis 4 C-Atomen, einen Alkenylrest mit 1 bis 4 C-Atomen oder einen gegebenenfalls substituierten Benzylrest oder einen $-CO-R^4$-Rest bedeutet, wobei $R^4$ einen gegebenenfalls durch Halogen oder Alkoxy substituierten Alkylrest mit 1 bis 4 C-Atomen oder einen aromatischen Rest bedeutet und $R^3$ ferner einen $-CO-NH-R^5$-Rest bedeutet, wobei $R^5$ einen Alkylrest mit 1 bis 4 C-Atomen oder einen aromatischen Rest bedeutet.

2. Mittel zur Beeinflussung des Pflanzenwachstums, enthaltend ein β-Triazolyloxim gemäss Anspruch 1.

3. β-Triazolyloxim, ausgewählt aus der Gruppe bestehend aus 3-(1′,2′,4′-Triazolyl-(1′))-1-phenyl-4,4-dimethyl-pentan-1-on-oxim, 3-(1′,2′,4′-Triazolyl-(1′))-1-(4″-fluor-phenyl)-4,4-dimethyl-pentan-1-on-oxim, 3-(1′,2′,4′-Triazolyl-(1′))-1-(4″-chlorphenyl)-4,4-dimethyl-pentan-1-on-oxim, 3-(1′,2′,4′-Triazolyl-(1′))-1-(4″-brom-phenyl)-4,4-dimethyl-pentan-1-on-oxim, 3-(1′,2′,4′-Triazolyl-(1′))-1-(4″-bromphenyl)-4,4-dimethyl-pentan-1-on-O-acetyloxim, 3-(1′,2′,4′-Triazolyl-(1′)-1-(4″-bromphenyl)-4,4-dimethyl-pentan-1-on-O-propionyloxim.

4. Mittel zur Beeinflussung des Pflanzenwachstums, enthaltend ein β-Triazolyloxim gemäss Anspruch 3.

5. Verfahren zur Beeinflussung des Pflanzenwachstums, dadurch gekennzeichnet, dass man die Pflanzen oder den Boden behandelt mit einem β-Triazolyloxim gemäss Anspruch 1.

**Claims for the Contracting States: BE, CH, DE, FR, GB, IT, LU, NL, SE**

1. A β-triazolyl oxime of the general formula

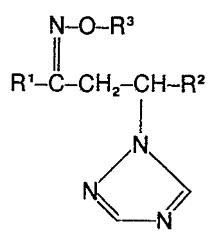

I

where

$R^1$ and $R^2$ are identical or different and each denotes alkyl of 1 to 6 carbon atoms, furanyl, thiophenyl, naphthyl, biphenylyl or phenyl, phenyl

being unsubstituted or substituted by fluoro, chloro, bromo, alkyl of 1 to 4 carbon atoms, alkoxy of 1 to 4 carbon atoms, trifluoromethyl or nitro, and $R^3$ denotes hydrogen, alkyl of 1 to 4 carbon atoms, alkenyl of 1 to 4 carbon atoms, unsubstituted or substituted benzyl, $-CO-R^4$, $R^4$ denoting alkyl of 1 to 4 carbon atoms which is unsubstituted or halogen- or alkoxy-substituted, or an aromatic radical, or $R^3$ denotes $-CO-NH-R^5$, $R^5$ denoting alkyl of 1 to 4 carbon atoms or an aromatic radical.

2. An agent for influencing plant growth, containing a β-triazolyl oxime as claimed in claim 1.

3. A β-triazolyl oxime selected from the group consisting of 3-(1′,2′,4′-triazolyl-(1′))-1-phenyl-4,4-dimethylpentan-1-one oxime, 3-(1′,2′,4′-triazolyl-(1′))-1-(4′′-fluorophenyl)-4,4-dimethylpentan-1-one oxime, 3-(1′,2′,4′-triazolyl-(1′))-1-(4″-fluorophenyl)-4,4-dimethylpentan-1-one oxime, 3-(1′,2′,4′-triazolyl-(1′))-1-(4″chlorophenyl)-4,4-dimethylpentan-1-on oxime, 3-(1′,2′,4′-triazolyl-(1′))-1-(4″-bromophenyl)-4,4-dimethylpentan-1-one oxime, 3-(1′,2′,4′-triazolyl-(1′))-1-(4″-bromophenyl)-4,4-methylpentan-1-one-O-acetyl oxime and 3-(1′,2′,4′-triazolyl-(1′))-1-(4′′-bromophenyl)-4,4-dimethyl pentan-1-one-O-propionyl oxime.

4. An agent for influencing plant growth, containing a β-triazolyl oxime as claimed in claim 3.

5. A process for influencing plant growth, characterized in that the plants or the soil are treated with a β-triazolyl oxime as claimed in claim 1.

**Revendications pour les Etats contractants: BE, CH, DE, FR, GB, IT, LU, NL, SE**

1. Un β-triazolyloxime de formule générale:

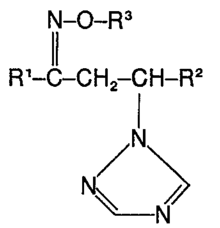

I

dans laquelle

$R^1$ et $R^2$ sont différents ou identiques et représentent un reste alkyle en $C_1$ à $C_6$, furanyle, thiophényle, naphtyle, biphénylyle ou phényle, le reste phényle pouvant être substitué par du fluor, chlore, brome ou un reste alkyle en $C_1$ à $C_4$, alcoxy en $C_1$ à $C_4$, trifluoro-méthyle ou nitro,

$R^3$ représente un atome d'hydrogène, un reste alkyle en $C_1$ à $C_4$, un reste alcényle en $C_1$ à $C_4$ ou un reste benzyle éventuellement substitué ou un reste $-CO-R^4$,

$R^4$ représentant un reste alkyle en $C_1$ à $C_4$, éventuellement substitué par un halogène ou un reste alcoxy, ou un reste aromatique et $R^3$ pouvant encore représenter un reste $-CO-NH-R^5$, avec $R^5$ représentant un reste alkyle en $C_1$ à $C_4$ ou un reste aromatique.

2. Moyen pour influencer la croissance des plantes comprenant un β-triazolyloxime selon la revendication 1.

3. Un β-triazolyloxime choisi dans le groupe constitué par 3-(1′,2′,4′-triazolyl-(1′))-1-phényl-4,4-diméthylpentan-1-on-oxime, 3-(1′,2′,4′-triazolyl-(1′))-1-(4′′-fluorophényl)-4,4-diméthyl-pentan-1-on-oxime, 3-(1′,2′,4′-triazolyl-(1′))-1-(4″-chlorophényl)-4,4-diméthyl-pentan-1-on-oxime, 3-(1′,2′,4′-triazolyl-(1′))-1-(4′′-bromophényl)-4,4-diméthylpentan-1-on-oxime, 3-(1′,2′,4′-triazolyl-(1′))-1-(4″-bromophényl)-4,4-diméthyl-pentan-1-on-O-acétyloxime, 3-(1′,2′,4′-triazolyl-(1′)-1-(4′′-bromophényl)-4,4-diméthyl-pentan-1-on-O-propionyloxime.

4. Moyen pour influencer la croissance des plantes comprenant un β-triazolyloxime selon la revendication 3.

5. Procédé pour influencer la croissance des plantes, caractérisé par le fait qu'on traite les plantes ou le sol avec un β-triazolyloxime selon la revendication 1.

**Patentansprüche für den Vertragsstaat: AT**

1. Mittel zur Beeinflussung des Pflanzenwachstums, enthaltend ein β-Triazolyloxim der allgemeinen Formel

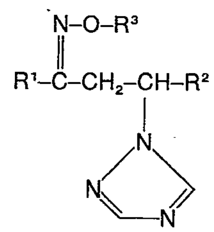

I

in der

$R^1$ und $R^2$ verschieden oder gleich sind und Alkyl mit 1 bis 6 C-Atomen, Furanyl, Thiophenyl, Naphthyl, Biphenylyl oder Phenyl bedeuten, wobei der Phenylrest durch Fluor, Chlor, Brom, Alkyl mit 1 bis 4 C-Atomen, Alkoxy mit 1 bis 4 C-Atomen, Trifluormethyl oder Nitro substituiert sein kann und

$R^3$ ein Wasserstoffatom, einen Alkylrest mit 1 bis 4 C-Atomen, einen Alkenylrest mit 1 bis 4 C-Atomen oder einen gegebenenfalls substituierten Benzylrest oder einen $-CO-R^4$-Rest bedeutet, wobei $R^4$ einen gegebenenfalls durch Halogen oder Alkoxy substituierten Alkylrest mit 1 bis 4 C-Atomen oder einen aromatischen Rest bedeutet und $R^3$ ferner einen $-CO-NH-R^5$-Rest bedeutet, wobei $R^5$ einen Alkylrest mit 1 bis 4 C-Atomen oder einen aromatischen Rest bedeutet.

2. Mittel zur Beeinflussung des Pflanzenwachstums, enthaltend ein β-Triazolyloxim, ausgewählt aus der Gruppe, bestehend aus 3-(1′,2′,4′-Triazolyl-(1′))-1-phenyl-4,4-dimethyl-pentan-1-on-oxim 3-(1′,2′,4′-Triazolyl-(1′))-1-(4″-fluor-phenyl)-4,4-dimethyl-pentan-1-on-oxim, 3-(1′,2′,4′-Triazolyl-(1′))-1-(4″-chlorphenyl)-4,4-dimethyl-pentan-1-on-oxim, 3-(1′,2′,4′-Triazolyl-(1′))-1-(4″-bromphenyl)-4,4-dimethyl-pentan-1-on-oxim, 3-(1′,2′,4′-Triazolyl-(1′))-1-(4″-bromphenyl)-4,4-dimethyl-pentan-1-on-O-acetyloxim, 3-(1′,2′,4′-Triazolyl-(1′)-1-(4″-bromphenyl)-4,4-dimethyl-pentan-1-on-O-propionyloxim.

3. Verfahren zur Beeinflussung des Pflanzenwachstums, dadurch gekennzeichnet, dass man

die Pflanzen oder den Boden behandelt mit einem Mittel gemäss Anspruch 1.

**Claims for the Contracting State: AT**

1. An agent for influencing plant growth, containing a β-triazolyl oxime of the general formula

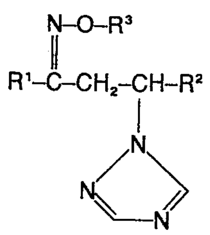

I

where

$R^1$ and $R^2$ are identical or different and each denotes alkyl of 1 to 6 carbon atoms, furanyl, thiophenyl, naphthyl, biphenylyl or phenyl, phenyl being unsubstituted or substituted by fluoro, chloro, bromo, alkyl of 1 to 4 carbon atoms, alkoxy of 1 to 4 carbon atoms, trifluoromethyl or nitro, and $R^3$ denotes hydrogen, alkyl of 1 to 4 carbon atoms, alkenyl of 1 to 4 carbon atoms, unsubstituted or substituted benzyl $-CO-R^4$, $R^4$ denoting alkyl of 1 to 4 carbon atoms which is unsubstituted or halogen- or alkoxy-substituted, or an aromatic radical, or $R^3$ denotes $-CO-NH-R^5$, $R^5$ denoting alkyl of 1 to 4 carbon atoms or an aromatic radical.

2. An agent for influencing plant growth, containing a β-triazolyl oxime selected from the group consisting of 3-(1′,2′,4′-triazolyl-(1′))-1-phenyl-4,4-dimethylpentan-1-one oxime, 3-(1′,2′,4′-triazolyl-(1′))-1-(4″-fluorophenyl)-4,4-dimethylpentan-1-one oxime, 3-(1′,2′,4′-triazolyl-(1′))-1-(4″-chlorophenyl)-4,4-dimethylpentan-1-one oxime, 3-(1′,2′,4′-triazolyl-(1′))-1-(4″-bromophenyl)-4,4-dimethylpentan-1-one oxime, 3-(1′,2′,4′-triazolyl-(1′))-1-(4″-bromophenyl)-4,4-dimethylpentan-1-one-O-acetyl oxime and 3-(1′,2′,4′-triazolyl-(1′))-1-(4″-bromophenyl)-4,4-dimethylpentan-1-one-O-propionyl oxime.

3. A process for influencing plant growth, characterized in that the plants or the soil are treated with an agent as claimed in claim 1.

**Revendications pour l'Etat contractant: AT**

1. Moyen pour influencer la croissance des plantes comprenant un β-triazolyloxime de formule générale

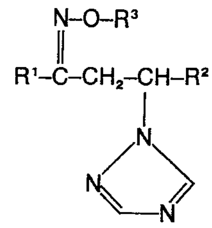

I

dans laquelle

$R^1$ et $R^2$ sont différents ou identiques et représentent un reste alkyle en $C_1$ à $C_6$, furanyle, thiophényle, naphtyle, biphénylyle ou phényle, le reste phényle pouvant être substitué par du fluor, chlore, brome ou un reste alkyle en $C_1$ à $C_4$, alcoxy en $C_1$ à $C_4$, trifluorométhyle ou nitro,

$R^3$ représente un atome d'hydrogène, un reste alkyle en $C_1$ à $C_4$, un reste alcényle en $C_1$ à $C_4$ ou un reste benzyle éventuellement substitué ou un reste $-CO-R^4$.

$R^4$ représentant un reste alkyle en $C_1$ à $C_4$, éventuellement substitué par un halogène ou un reste alcoxy, ou un reste aromatique et $R^3$ pouvant encore représenter un reste $-CO-NH-R^5$, avec $R^5$ représentant un reste alkyle en $C_1$ à $C_4$ ou un reste aromatique.

2. Moyen pour influencer la croissance des plantes comprenant un β-triazolyloxime choisi dans le groupe constitué par 3-(1′,2′,4′-triazolyl-(1′))-1-phényl-4,4-diméthyl-pentan-1-on-oxime, 3-(1′,2′,4′-triazolyl-(1′))-1-(4″-fluorophényl)-4,4-diméthyl-pentan-1-on-oxime, 3-(1′,2′,4′-triazolyl-(1′))-1-(4″-chlorophényl)-4,4-diméthyl-pentan-1-on-oxime,3-(1′,2′,4′-triazolyl-(1′))-1-(4″-bromophényl)-4,4-diméthyl-pentan-1-on-oxime, 3-(1′,2′,4′-triazolyl-(1′))-1-(4″-bromophényl)-4,4-diméthylpentan-1-on-O-acétyloxime, 3-(1′,2′,4′-triazolyl-(1′)-1-(4″-bromophényl)-4,4-diméthyl-pentan-1-on-O-propionyloxime.

3. Procédé pour influencer la croissance des plantes, caractérisé par le fait qu'on traite les plantes ou le sol avec un moyen selon la revendication 1.